# EUROPEAN PATENT SPECIFICATION

(11) **EP 1 727 473 B1**
(45) Date of publication and mention of the grant of the patent: **28.03.2012**
(21) Application number: 05717773.5
(22) Date of filing: 23.02.2005
(51) Int. Cl.: A61B 10/00, A61B 10/02, A61F 13/38, B01L 3/00, G01N 1/02

(54) **Diagnostic swab and biopsy punch systems**
Diagnostische Abstrich- und Biopsiestanzsysteme
Systèmes d'écouvillon et de biopsie pour diagnostic

(30) Priority: 23.02.2004 GB 0403976; 23.02.2004 GB 0403978; 15.07.2004 US 587861 P; 28.01.2005 GB 0501818
(43) Date of publication of application: 06.12.2006
(73) Proprietor: ETHICON, INC., Somerville, NJ 08876-0151 (US)
(72) Inventor: BAYLIFF, Simon, William, Skipton, North Yorkshire BD23 6AH (GB); DEL BONO, Michelle, Barnoldswick, Lancashire BB18 5HJ (GB); BIOTT, Maurice, Charles, Keighley, West Yorkshire BD20 9LT (GB); LINDSAY-WATT, Sylvia, Steeton, West Yorkshire BD20 6UN (GB)
(74) Representative: James, Anthony Christopher W.P.
(86) International application number: PCT/GB2005/000680
(87) International publication number: WO 2005/082254

(56) References cited:
- WO-A-2005/044110
- US-B1- 6 524 530

## Description

### FlELD OF THE INVENTION

The present invention relates to diagnostic swab and biopsy punch test devices comprising diagnostic caps.

### TECHNICAL BACKGROUND OF THE INVENTION

Absorbent swabs are generally known in the medical arts for use in collecting fluid specimens from a patient for further analysis. Medical swabs commonly comprise a fibrous swab tip at one end of an elongated stick or shaft which is manually handled to contact the swab tip with a selected part of a patient, for example the surface of a wound. As a result, some tissue fluid, including cellular matter, adheres to the swab tip which can then he contacted with one or more selected reagents to indicate the presence of infection or other information regarding patient condition. Tests commonly performed with swab specimens include fluorescent tests, enzymatic tests, monoclonal antibody based tests and agglutination tests.

Still greater diagnostic accuracy can sometimes be achieved by analysis of a biopsy sample. Typically, the biopsy sample may be taken by means of a cylindrical, sharpened biopsy punch located at one end of an elongated stick or shaft which is manually punched into the tissue of interest. The punch sample is then homogenized and analysed with suitable reagents to arrive at a diagnosis.

In accordance with standard techniques, the collected biological specimen (swab or biopsy) is normally transferred from the swab tip or the biopsy punch to a slide or other laboratory apparatus such as a test tube or the like for contact with selected reagents and further analysis. However, it can be difficult to ensure transfer of a sufficient specimen quantity from the swab tip to the laboratory slide or test tube to ensure accurate test results. Contamination of the sample can accidentally take place during the transfer, and delays between the time of specimen colleclion and actual test performance can also result in a decrease in test reliability. The need for a separate analysis step also increases the overall cost of the diagnostic procedure.

US-A-5266266 describes a diagnostic swab having a hollow swab shaft extending between u swab tip adapted to collect a targeted specimen and a break-off nib that protrudes into a reservoir of reagent solution. Following collection of a sample on the swab tip, deformation of the reservoir is effected to sever the nib from the swab to open the rear end of the swab shaft and permit reagent flow from the reagent chamber through the swab shaft to the swab tip. The swab is fully enclosed in a housing having a cap, in which may be provided a further reagent, such as treated beads, for reaction with the eluate from the swab tip. This swab arrangement requires quite large amounts of thermoplastic molding material for its construction, and quite large amounts of reagent solution for satisfactory operation, with resulting increased cost and loss of sensitivity due to dilution of the swab sample.

US-A-6248294 describes a self-contained diagnostic swab arrangement comprising a conventional swab and a diagnostic housing to receive and enclose the swab after collection of a sample. The diagnostic housing includes a reservoir of reagent liquid, and a diagnostic test strip extending up one side of the housing, This diagnostic swab arrangement requires quite large amounts of thermoplastic molding material for its construction, and an excessive amount of reagent solution for satisfactory operation.

US-B-6524530 and WO-A-2005044110 (which forms part of the state of the art by virtue of Article 54(3) EPC) described further diagnostic swab arrangements having elongated caps containing diagnostic test reagents that are fitted over the swab for analysis of samples collected by the swab.

### SUMMARY OF THE INVENTION

The present invention provides a diagnostic test apparatus according to claim 1.

The use of a shaft that is adapted in this way to enable fitting of a cap near to the first end allows the use of a small diagnostic cap with the apparatus. This provides for economy of materials usage. Extremely compact diagnostic measurement and display configurations can be fitted inside such a small cap, thereby minimizing the overall size of the apparatus, reagent usage, and sample dilution, as will be made clear in the following discussion.

Suitably, the cap comprised a sample receiving port having an interior surface adapted for engagement with the cap engagement elements on the shaft, and a housing defining a lateral flow path for a liquid sample, the lateral flow path having an inlet end in fluid communication with the sample receiving port

Suitably, the test apparatus according to the invention comprises both a first said shaft having a swab attached thereto, and a second said shaft having a biopsy punch attached thereto, and wherein the diagnostic cap can be secured interchangeably on the said first and second shalls.

### DETAILED DESCRTPTTON OF THE INVENTION

### Swab/Biopsy Punch and Shatf

The cap engagement element on the shaft for fitting of the cap is located from 1mm to about 30mm from the tip of the swab or the biopsy punch. This is consistent with the use of the relatively compact diagnostic caps described,

The cap engagement element on the shaft for fitting of the cap may be a tapered region of the shaft for forming an interference fit with the cap, for example it may appear as a truncated cone that is coaxial with the shaft and tapers towards the first end of the shaft. Or the whole shaft may have a diameter larger than that of the swab or biopsy punch, with a tapered region adjacent to the first end. In any case, the diameter of the tapered region where it engages with the cap is greater than the diameter of the swab or biopsy punch, so that the cap can be fitted over the swab or biopsy punch.

In other embodiments, the cap engagement element may comprise a snap-fitting projection for forming a snap-fit with one or more complementary projections on an inner surface of the cap, or a threaded projection for forming a screw fit with one or more complementary threads on an inner surface of the cap. Quick-fit threads such as bayonet threads or a Luer lock are especially suitable for use as the engagement elements on the cap and the shaft.

The swab may be any absorbent swab, for example a nonwoven fibrous swab. Typically the diameter of the swab is about 2 to about 5mm, for example about 3mm. In certain embodiments, the swab may be formed from a medically acceptable open-celled foam, for example a polyurethane foam, since such foams have high absorbency and can readily be squeezed to expel absorbed fluids. The biopsy punch will typically be a stainless steel cylindrical punch of diameter about 1mm to about 10mm, for example about 3mm to about 8mm, suitably about 6mm.

In certain embodiments the shaft is hollow, whereby a fluid can be passed down the shaft from the second end to expel a biological sample from the swab or the biopsy punch into the cap for diagnostic analysis. The shaft may comprise a fitting at the second end for attachment of a syringe or other source of the fluid. In certain embodiments, the apparatus may comprise a reservoir of liquid attached to the second end of the shaft, for example a compressible bulb containing the liquid, which can be activated after use of the swab or biopsy punch. Suitable devices of this kind are described, for example in US-A-5266266, the entire content of which is incorporated herein by reference. In other embodiments, the apparatus may comprise a plunger that can be pushed down the hollow bore of the shaft to expel fluid or other specimens from the swab or biopsy punch.

Another advantage of the hollow shaft is that, where the apparatus is a biopsy punch, the biopsy sample can more readily be pushed or blown out of the punch. The biopsy punch apparatus can further comprise a homogenizing tool that can be passed down the hollow shaft to homogenize a tissue sample in the biopsy punch. This step of homogenizing can be followed, if necessary, by passing liquid down the shaft from the second end to expert the homogenized tissue from the biopsy punch into the cap for diagnostic analysis.

### Cap Construction - General

The diagnostic cap in the apparatus of the present invention comprises, and may consist essentially of, a body portion (sample receiving port) generally in the shape of a cup, that is to say a short tube open at one end, for receiving the swab or biopsy punch.

Suitably, the cap body portion (sample receiving port) has a length (measured along the axis of the shaft when the cap is secured on the shaft) ot from about 1cm to about 4cm, for example from about 15mm to about 25mm. Suitably, the cap body portion (sample receiving port) has an internal diameter of from about 1 mm to about 10 mm, for example from about 2mm to about 6mm. It follows that the internal volume of the cap (and of the sample receiving port when it is secured on the shaft is small, suitably from about 10mm³ to about 1000mm³, for example from about 50mm³ to about 300mm³. This small volume permits diagnostic tests to be carried out on small samples, for example with litlle or no dilution of samples collected by a typical swab or biopsy punch.

An internal surface of the cap body is preferably provided with cap fitting elements for securing the cap to the shaft. These may comprise a snap-fitting projection for forming a snap-fit with one or more complementary projections on an outer surface of the shaft, or a threaded projection for forming a screw fit with one or more complementary threads on an outer surface of the shaft. Quick-fit thread elements, such as bayonet threads or a Lucr luck are especially suitable for use as the engagement elements on the cap and the shaft. the complementary fitting elements on the cap and the shaft are preferably adapted to provide a substantially liquid-tight seal between the cap and the shaft. This ensures that escape of liquid sample from the sample receiving port of the cap takes place by liquid passing through the cap to an outlet that is downstream of one or more diagnostic test materials.

A tapered projection may extend upwardly from a base of the cap body towards the opening of the cap into which the swab or biopsy punch is inserted. In certain embodiments, the tapered projection may have a sharpened, abrasive or serrated surface for macerating a tissue sample in a biopsy punch inserted into the cap.

Suitably, the cap is at least partially transparent. This allows visible diagnostic indicators to be observed through the cap without removing the cap. In some embodiments the cap is at least partially transparent to ultraviolet light, for example light having wavelength 300-350 nanometers. In these or other embodiments, there may be window apertures in the cap to permit observation of a diagnostic indicator inside the cap.

The caps contain compact diagnostic test devices that can fit within the small caps hereinbefore described, and that can be used to analyse one or more diagnostic indicators, especially in samples having a small volume.

In certain embodiments the caps contain at least one diagnostic test reagent provided in or around an absorbent plug locatcd within the body of the cap. Suitably, the absorbent plug has an uncompressed volume of from about 10 to about 1000mm³, for example from about 50 to about 300 mm³. The absorbent plug effectively wicks the analyte solution to the diagnostic indicators in the cap body.

For example, in some embodiments the at least one diagnostic test reagent is provided in or on an annular diagnostic strip extending at least part of the way around the inside of the cap body, and preferably the whole way around the inside of the cap body. The strip (or strips) may be segmented into a plurality of regions adapted to detect different diagnostic indicators, whereby these regions are radially spaced around the inside of the cap body. The strip (or strips) may be wrapped around an absorbent plug located within the body of the cap, whereby the absorbent plug wicks the fluid under test to the strip or strips. It has been found that an especially suitable plug for this kind of wicking is provided by a cylindrical bundle of hydrophilic fibers, such as hydrophilic polyester fibers.

In other embodiments at least one diagnostic test reagent is provided in or on a diagnostic sheet extending transversely across the inside of the cap body. The edges of the sheet appear then as a ring when viewed through the side walls of the cap body. A stack of such sheets may make up a plug inside the cap body, and different sheets in the stack may be adapted to indicate the presence of different biological markers. Preferably, the sheets are made of absorbent material such as filter paper, so as to draw fluid sample from the swab or the biopsy punch.

In the above embodiments, the diagnostic cap body itself (e.g. the sample receiving port) may bear radially and/or axially spaced indicia corresponding to the different regions or layers of diagnostic material inside the cap.

In the above embodiments a venting aperture may be provided in the base of the cap body. This assists smooth flow of the biological sample from the swab or biopsy punch into the cap body for diagnostic testing.

In certain embodiments, the diagnostic cap consists essentially of the cup-shaped body component. These embodiments are especially compact and economical of materials and sample usage.

### Lateral Flow

In the lateral-flow diagnostic cap embodiments, the cap suitably comprises a sample receiving port having an interior surface adapted for engagement with the cap engagement elements on the shaft, and a housing defining a fluid flow path for a liquid sample, the fluid flow path having an inlet end in fluid communication with the sample receiving port

The size, shape and configuration of the sample receiving port are preferably as hereinbefore described. In some embodiments the housing preferably further comprises a base for the cap, whereby the cap can rest on said base on a horizontal surface with the sample receiving port opening in an upward direction to receive the shaft. Preferably, the axis of the sample receiving port is vertical or near-vertical (e.g. within about 30° of verlical), and in any case the base and port arc configured so that the swab or biopsy punch can remain upright when secured in the sample receiving port. This is advantageous as it allows the sample to drain out of the swab or biopsy punch into the diagnostic device under the influence of gravity, simply by standing the device on the cap base.

Suitably, the housing has a substantially flat lower surface forming the base for the housing, and the sample receiving port opens upwardly from an upper surface of the housing opposite said flat lower surface. For example, the base may be a substantially laminar structure with the sample receiving port projecting upwardly therefrom, preferably front a central region thereof to provide maximum stability when the swab shaft is received in the sample receiving port.

It follows that the fluid flow path preferably extends substantially in a plane that is substantially perpendicular to the axis of the sample receiving port. The term "substantially perpendicular" generally indicates a plane having a normal axis within about 30° of the axis of the sample receiving port. This allows the side walls of the flow path to form at least part of the base of the housing perpendicular to the axis of the sample receiving port as described above.

The fluid flow path of the assay device will typically be capable of supporting lateral flow of aqueous liquids. By "lateral flow", it is meant liquid flow in which an of the dissolved or dispersed components of the liquid are carried, preferably at substantially equal rates, and with relatively unimpaired flow, laterally through the carrier. Suitably, the fluid flow path contains one or more porous carrier materials. The porous carrier materials are preferably in fluid communication along substantially the whole fluid flow path so as to assist transfer of fluid along the path by capillary action. Suitably, the porous carrier materials are hydrophilic, but preferably they do not themselves absorb water. The porous carrier materials may function as solid substrates for attachment of the reagent moieties in the reaction zone and/or the detector moieties in the detection zone.

Suitable materials for forming the porous carrier materials include any suitable natural or synthetic polymer, including insoluble polysaccharides such as cellulose, and synthetic polymers such as polyacrylates, high density porous polyethylene sheet materials, polyvinyl chloride, polyvinyl acetate, copolymers of vinyl acetate and vinyl chloride, polyamide, polycarbonate, nylon, glass fiber, orlon, polyester, polystyrene, and mixtures and combinations thereof. A suitable material is POREX (Registered Trade Mark) lateral flow membrane, available from Porex Technologies Corp., Fairburn Georgia, USA. Suitably, the carrier material comprises pendant amine, ester or carboxylate groups to assist conjugation.

The diagnostic cap may comprise a reaction zone in the fluid flow path comprising a reagent moiety that reacts with an analyte in wound fluid to release an indicator moiety; and a detector moiety located in a detection zone downstream from the reaction zone in the fluid flow path, wherein the detector moiety can interact with the indicator moiety to produce a detectable change in the detection zone. The reagent moiety and/or the detector moiety may at least initially be bound to a suitable solid carrier material and thereby immobilized.

The size and shape of the carrier are not critical and may vary. The carrier defines a lateral flow path. Suitably, the porous carrier is in the form of one or more elongate strips or columns. In certain embodiments, the porous carrier is one or more elongate strips of sheet material, or a plurality of sheets making up in combination an elongate strip. The reaction zone and detection zone would then normally be spaced apart along the long axis of the strip. However, in some embodiments the porous carrier could, for example be in other sheet forms, such as a disk. In these cases the reaction zone and detection zone would normally be arranged concentrically around the center of the sheet, with a sample application zone in the center of the sheet. In yet other embodiments, the carrier is formed of carrier beads, for example beads made from any of the materials described above. The beads may suitably be sized from about 1 micrometer to about 1mm. The beads may be packed into the flow path inside the housing, or may be captured or supported on a suitable porous substrate such as a glass fiber pad..

Normally, the fluid flow path further comprises a sample application zone upstream of the reaction zone and in fluid communication with the inside of the cap body. The device is configured such that, in use, the fluid sample flows laterally from the sample application zone to the reaction zone and then to the detection zone.

It will be appreciated that the devices according to the present invention may be adapted to detect more than one analyte. This can be done by the use of several different reagent moieties in a single reaction zone, or preferably by the provision in a single device of a plurality of lateral flow paths each having a different reagent moiety in its respective reaction zone for detecting a different analyte., for example the plurality of lateral flow paths may be radially distributed around a cap body. In some embodiments, the plurality of fluid flow paths are physically separated by the housing. In other embodiments multiple lateral flow paths (lanes) can be defined in a single lateral flow membrane by depositing lines of wax or similar hydrophobic material between the lanes.

Suitably, the plurality of lateral flow paths are defined as separate fluid flow paths in the housing, and preferably the plurality of lateral flow paths are spaced radially around the sample receiving port in a plane substantially perpendicular to the axis of the sample receiving port. The housing of the plurality of radially spaced flow paths can then provide a stable base extending radially around the sample receiving port.

The housing is normally made of a thermoplastic material, preferably at least part of the housing in molded in one piece with the cap body. Suitable materials include polyethylene, polypropylene, polyacrylate, polyamide or polystyrene. Suitably, the housing is made by fitting together two plastic components forming the top and bottom of the housing, respectively. Suitable methods for making the housing components include injection molding and stereolithography. Suitably, the housing is at least partially transparent. This allows visible diagnostic indicators to be observed through the housing. In some embodiments the housing is at least partially transparent to ultraviolet light, for example light having wavelength 300-350 nanometers. In some embodiments, the housing comprises a window for observing the detection zone. .

In all of the above embodiments, when the cap is secured on the shaft, the swab or the biopsy is preferably in contact with an absorbent material that can wick fluid from the swab or the biopsy to the one or more diagnostic reagents. This minimizes the need for a diluent liquid to wash the sample out of the swab or biopsy into the diagnostic material, as required in the prior art hereinbefore described. In certain embodiments, the diagnostic cap is shaped and configured to compress the swab when the cap is secured to the shall, thereby to squeeze liquid sample out of the swab and into the diagnostic materials. This embodiment is especially suitable where the swab comprises an open-celled foam material.

Suitably, the cap may be provided with a filter that is located between the swab or biopsy punch and the diagnostic material when the cap is secured on the shaft in use. The filter may be any paper or microporous film suitable for separating solid debris from the analyte solution to be passed to the diagnostic material.

Suitably, the cap may be provided with a fill indicator. That is to say, a means to indicate when the diagnostic material or materials have all been wetted by the analyte solution. For example, the fill indicator could be a sheet of filter paper in the base of the cap, or at the distal end of a lateral flow path, that has been treated to change color when wetted by the analyte solution.

### Diagnostic Chemistry

At least a portion of the diagnostic material in the cap preferably undergoes a color change (the term color change includes chemiluminescence and/or a change in appearance under UV light) in the presence of one or more analytes, and preferably this color change is visible through the side wall of the cap.

Analytes that could be detected by the diagnostic caps according to the present invention include, but are not limited to, the group selected from pH, redox potential, free radical activity, activated oxygen, Fe³⁺; protease enzymes such as those discussed in more detail below; other enzymes such as lysozyme, acid hydrolases, lactate dehydrogenase, glycosidases, cathepsins B, L, D, G, plasmin, and plasminogen activator; indicators of wound infections such as lipopolysaccharides, in particular phospholipase A protein, or outer membrane proteins, such as omp T; protease inhibitors such as TIMPs, PAIs, alpha I antitrypsin, macroglobulin; cytokines such as TNFalpha; Interleukins such as IL-1, Il-6,IL-10; growth factors including GM-CSF, VEGF, PDGF,TGF-beta, IGF; soluble growth factor receptors; cytokine antagonists; soluble VEGF receptor IL-1receptor antagonist; matrix components or fragments thereof, such as glycosaminoglycans including hyaluronic acid, collagen peptides, fibronectin fragments, fibrin (ogen) fragments; cell surface receptors (especially for tissue biopsy) such as CD44, Integrins, PDGF-receptor, plasminogen/ u-PA receptors; and chemotractant molecules including leukotriene B4, C5A, and formylated peptides.

The analyte may be an enzyme. For example, the enzyme may be selected from the group consisting of mammalian host-derived enzymes and microbial enzymes. The term "mammalian host-derived enzyme" refers to an endogenous enzyme found in mammalian wound fluid. The term "microbial enzymes" includes protease enzymes that have been secreted by microorganisms, or expressed on the cell surface of microorganisms such as bacteria or fungi. The enzyme may be a protease enzyme. The term "protease enzyme" refers to an enzyme that specifically or non-specifically cleaves a peptide, polypeptide, or protein.

Endogenous (i.e. host-derived) protease enzymes that may be detected by the devices and systems of the present invention are suitably selected from the group consisting of matrix metalloproteinases (MMP's), elastase, stromelysin, kallikrein and thrombin. Suitably, the endogenous protease is selected from the group consisting of neutrophil proteases and macrophage proteases. Preferred protease enzymes include collagenases (e.g. MMP-1 and MMP-8), gelatinases (e.g. MMP-9) and neutrophil elastase, MMP-2, MMP-12, proteinase 3, plasmin, low molecular weight gelatinases and latent or active elastases, interleukin converting enzymes and tumor necrosis factor (TNFα) converting enzymes.

Examples of pathogenic bacteria that may be detectable by the present invention include, but are not limited to staphylococcus (for example, Staphylococcus aureus, Staphylococcus epidermidis, or Staphylococcus saprophyticus), streptococcus (for example, Streptococcus pyogenes, streptococcus pneumoniae, or Streptococcus agalactiae), enterococcus (for example, Enterococcus faecalis, or Enterococcus faecium), corynebacteria species (for example, Corynebacterium diptheriae), bacillus (for example, Bacillus anthracis), listeria (for example, Listeria monocytogenes), Clostridium species (for example, Clostridium perfringens, Clostridium tetanus, Clostridium botulinum, Clostridium difficile), Neisseria species (for example, Neisseria meningitidis, or Neisseria gonorrhoeae), E. coli, Shigella species, Salmonella species, Yersinia species (for example, Yersinia pestis, Yersinia pseudotuberculosis, or Yersinia enterocolitica), Vibrio cholerae, Campylobacter species (for example, Carnpylobacter jejuni or Campylobacter fetus), Helicobacter pylori, pseudomonas (for example, Pseudomonas aeruginosa or Pseudomonas mallei), Peptostreptococcus (for example Peptostreptococcus magnus), Bacteroides fragilis, Haemophilus influenzae, Bordetella pertussis, Mycoplasma pneumoniae, Ureaplasma urealyticum, Legionella pneumophila, Treponema pallidum, Leptospira interrogans, Borrelia burgdorferi, mycobacteria (for example, Mycobacterium tuberculosis), Mycobacterium leprae, Actinomyces species, Nocardia species, chlamydia (for example, Chlamydia psittaci, Chlamydia trachomatis, or Chlamydia pneumoniae), Rickettsia (for example, Rickettsia ricketsii, Rickettsia prowazekii or Rickettsia akari), brucella (for example, Brucella abortus, Brucella melitensis, or Brucella suis), Proteus mirabilis, Serratia marcescens, Enterobacter clocae, Acetinobacter anitratus, Klebsiella pneumoniae and Francisella tularensis.

In certain embodiments, the microbial enzyme analyte comprises a bacterial protease selected from the group consisting of bacterial enzymes of *Pseudomonas Aeruginosa, Enterococcus faecalis, Escherichia Coli, Streptococcus Pyogenes* and *Staphylococcus Aureus.* The enzymes are species specific and in some cases the actual enzyme marker being detected is not known.

In suitable embodiments, the diagnostic material in the apparatus of the present invention comprises a pH-indicator or a redox indicator. Low pH and high oxidative stress are both characteristics of infected or chronic wounds. Colorimetric pH indicators bound to solid substrates, such a universal indicator papers, are well known in the art. Redox indicators include substances that undergo a color change in the presence of reactive oxygen species such as superoxide or hydroxyl radicals. One such indicator molecule is diphenylpicrylhydrazyl, which undergoes a color change from purple to colorless in the presence of free radicals.

In suitable embodiments, the diagnostic material in the apparatus to the present invention contains one or more immunological binding partners to bind the one or more analyte molecules present in the sample. The immunological binding partners may for example comprise monoclonal or polyclonal antibodies, antibody fragments, or chimeric antibodies. Alternatively, the immunological binding partners may comprise antigens in cases where the presence of predetermined antibodies in the sample is being mapped. Preferably, the immunological binding partners comprise monuclonal antibodies. Preferably, the immunological or other binding partners are immobilised on a solid carrier material, for example by avidin-biotin linking, or dialdehyde derivatization of the support (carrier) material, followed by cross-linking to a peptide binding partner. Other immunological binding partners and/of reagents or indicator molecules may be present in the solution optionally used to expel the sample from the swab or biopsy punch as hereinbefore described.

The solid support materials immunological or other binding partners may be used in a range of immunoassays to map the presence of biologically active molecules. For example, the support having antibodies or antibody fragments bound thereto may be used in sandwich immunoassay-type mapping. Alternatively, the support may have analog ligands bound to the antibodies, whereby the molecules present in the wound fluid arc detected by affinity displacement immunoassay. Various other immunoassays will be apparent to persons skilled in the art.

Lateral flow immunoassay devices are described, for example, in WO95/04280.

As already noted, the analytes of interest may include enzymes that can modify substrates, for example proteins or polypeptides, by cleavage. Such modification of peptide substrates can be detected to determine the presence or absence of the analyte in a sample. Accordingly, in suitable embodiments, the diagnostic material in the apparatus of the present invention comprises a chemiluminescent, chromogenic or fluorogenic substrate for an enzyme analyte present in the sample.

One method for detecting the modification of a substrate by an enzyme is to label the substrate with two different dyes, where one dye serves to quench the fluorescence of the other dye by fluorescence resonance energy transfer (FRET) when the dye molecules are in close proximity. A typical acceptor and donor pair for resonance energy transfer consists of 4-[[-(dimethylamino)phenyl]azo]benzoic acid (DABCYL) and 5-[(2-aminoethylamino] naphthalene sulfonic acid (EDANS). EDANS is excited by illumination with 336 nanometer light, and emits a photon with a wavelength of 490 nanometers. If a DABCYL moiety is located within 2 nanometers of the EDANS, this photon will be efficiently absorbed. DABCYL and EDANS can be attached to opposite ends of a peptide in the diagnostic material used in the systems of the present invention. If the peptide is intact, FRET will be very efficient. If the peptide has been cleaved by an enzyme analyte, the two dyes will no longer be in close proximity and FRET will be inefficient. The cleavage reaction can be followed by observing either a decrease in DABCYL fluorescence or an increase in EDANS fluorescence (loss of quenching).

Another suitable diagnostic material comprises a chromogenic dye conjugated to a solid support by a suitable cleavable substrate moiety, such as a peptide. The chromogenic dye will change color when the linker group is cleaved by the analyte of interest. For example, para-nitrophenyl is colorless when linked to the support, and turns yellow when cleaved. The analyte concentration can be determined by measuring absorbance at 415 nanometers. Other dyes that produce detectable color change upon cleavage are known to those skilled in the art.

In yet another embodiment, the diagnostic material may comprise a colored support having a differently-colored molecule conjugated thereto by a linker moiety that can be cleaved by an analyte, for example an enzyme in the sample, Cleavage of the dye from the colored support can thereby result in a color change of the diagnostic material.

The solid support material used for the above identified assays of enzyme activity and immuno-assays may comprise any suitable solid material as discussed above. The cleavable cross-linkages where present generally comprise cleavable oligopeptidic sequences or eleavable oligosaccharides, each typically of twenty residues or fewer, for example from 3 to 15 residues.

The sensitivity of the diagnostic material will depend on a number of factors, including the length of the cleavable linker sequences. Steric hindrance may also he reduced by compling the cleavable oligopeptidie-sequence to the polymer by means of an appropriate spacer. Thus, the oligopeptidie sequences may couple the polymers directly (in which case the cross-linkage consists of the oligopeptidic sequence) or by means of an appropriate spacer. Suitable conjugation methods incorporating spacers are described in US-A-5770229.

The following, paper gives a useful review of bioconjugation techniques for use in pharmaceutical chemistry: Veronese, F.M. and Morpurgo, M (1999) Bioconjugation in Pharmaceutical chemistry Il Farmaco, 54, 497-516 and Ulbrich, K., et al (2000) Journal of controlled release 64, 63-79. .

The present invention is especially suitable for detection of a wide variety of enzymes in biological samples. Typically, the enzyme is selected such that elevated levels of the enzyme in a wound fluid are associated with pain, wound infection or wound chronicity. Usually, the enzyme is a protease, and the linker group comprises an oligopeptidic sequence which is a substrate for the protease.

In certain embodiments, the proteases to be detected may include elastase. Elastase levels are elevated in a range of wound healing disorders, including infected wounds and chronic wounds. In such embodiments, suitable substrate linkers may include one or more of the oligopeptidic sequences lys-gly-ala-ala-ala-lys -Ala-Ala-Ala-, Ala-Ala-Pro-Val, Ala-Ala-Pro-Leu, Ala-Ala-Pro-Phe, Ala-Ala-Pro-Ala or Ala-Tyr-Leu-Val.

In certain embodiments, the proteases to be detected may include a matrix metalloproteinase, in particular MMP-2 or MMP-9. These matrix metalloproteinases are elevated in chronic wounds such as venous ulcers, diabetic ulcers and pressure sores. In these embodiments, the cleavable linker may comprise the oligopeptidic sequence -Gly-Pro-Y-Gly-Pro-Z-,-Gly-Pro-Leu-Gly-Pro-Z-,-Gly-Pro-Ile-Gly-Pro-Z-, or-Ala-Pro-Gly-Leu-Z-, where Y and Z are amino acids.

In certain embodiments, the proteases to be detected may include a collagenase. Collagenase is elevated in chronic wounds such as venous ulcers, diabetic ulcers and pressure sores. In these embodiments, the cleavable linker may comprise the oligopeptidic sequence -Pro-Leu-Gly-Pro-D-Arg-Z-, -ProLeu-Gly-Leu-Leu-Gly-Z-,-Pro-Gln-Gly-Ile-Ala-Gly-Trp-,-Pro-Leu-Gly-Cys (Me)-His-,-Pro- Leu-Gly-Leu-Trp-Ala-,-Pro-Leu-Ala-Leu-Trp-Ala-Arg-, or-Pro-Leu-Ala-Tyr-Trp-Ala-Arg-, where Z is an amino acid.

In certain embodiments, the proteases to be detected may include a gelatinase. Gelatinase is elevated in chronic wounds such as venous ulcers, diabetic ulcers and pressure sores. In these embodiments, the cleavable linker may comprise the oligopeptidic sequence -Pro-LeuGly-Met-Trp-Ser-Arg-.

In certain embodiments, the proteases to be detected may include thrombin. In these embodiments, the cleavable linker may comprise the oligopeptidic sequence -Gly-Arg-Gly-Asp-, -Gly-Gly-Arg-, -Gly-Arg-Gly-Asp-Asn-Pro-, -Gly-Arg-Gly-Asp-Ser-, -Gly-Arg-Gly-Asp-Ser-Pro-Lys-,-Gly-Pro-Arg-, -Val-Pro-Arg-, or-Phe-Val-Arg-.

In certain embodiments, the proteases to be detected may include stromelysin. In these embodiments, the cleavable linker may comprise the oligopeptidic sequence -Pro-TyrAla-Tyr-Trp-Met-Arg-.

In certain embodiments, the proteases to be detected may include a kallikrein. The term "a kallikrein" refers to all serine proteases, whose activation is associated with the degradation of kininngen to form kinins, which are implicated in the onset of pain. Suitable peptide sequences for use in cleavable substrate for kallikrein include -Phe-Arg-Ser-Ser Arg-Gln or -Met-Ile-Ser-Leu-Met-Lys-Arg-Pro-Glu- that can be degraded by kallikrein at Lys-Arg or Arg-Ser bonds.

In addition to the proteases, it is also envisaged that the enzyme could be, fur example, an antibacterial chitinase or chitosanase such as lysozyme (elevated in infected wounds), in which case the substrate for the enzyme would be a polysaccharide or oligosaccharide comprising, D-glucosamine or N-acetyl D-glucusamine residues.

The peptide linkers for the detection of specific microbial protease enzymes are determined using a variety of techniques including literature references tn the natural substrate of the enzyme and empirical screening. Methods of identifying bacterial enzymes and peptide substrates therefor are described in WO03/063693, US-A-2003/0096315 and WO2004/047614. Suitable peptide linkers include the following:
Sequence 1: ETKVEENEAIQK
Sequence 2: VTLENTALRC
Sequence 3: QADALHDQASALKC
Sequence 4: KVSRRRRRGGDKVDRRRRRGGD

Sequences 1 and 2 above are specific for *Staphylococcus,* Sequence 3 is specific for *Pseudomonas,* Sequence 4 is specific to *E*. *coli,*

In some embodiments, the peptide is u sequence having al least 50%, 60%, 70%, 80%, 85%, 90%, 95%, or 99% sequence identity to one of the sequences listed herein, as determined using a sequence comparison program and parameters described herein.

In the lateral flow embodiments, the indicator moiety may comprise an indicator enzyme, and the detection zone then preferably contains a reagent that undergoes a visible change in the presence of the indicator enzyme. For example, the indicator enzyme may be a redox enzyme. Suitable indicator enzymes include laccase (CotA), green fluorescent protein (GFP), luciferase, alkaline phosphatase (ALP), p-galactosidase, acetylcholinesterases, and in particular horseradish peroxidase (HRP).

The detector moiety in the detection zone preferably undergoes a color change (the term color change includes chemiluminescence and/or a change in appearance under UV light) in the presence of one or more of the indicator moieties, and preferably this color change is visible through the window or side wall of the housing. In these and other embodiments of the invention, the color change may be assessed either visually (e.g. by comparison with a color key) or by optical detection devices, such as reflectance analyzers, video image analyzers and the like. Thus, target analyte levels may be determined by devices of the present invention.

Suitably, the indicator moity is a redox enzyme, and in these embodiments the reagent in the detection zone may then be a redox indicator. For example, where the indicator moiety is HRP, the indicator moiety may comprise 4-chloro-1-naphthol, which undergoes a color change from colorless to blue in the presence of HRP and hydrogen peroxide. It is also possible that other HRP substrates could be used. These would include TBM (3,3,5,5-tetramethylbenzidine), DAB (3,3-Diaminobenzidine), DAB with metal enhancement such as cobalt and nickel, AEC (3-amino-9 ethylcarbazole), or a combination of these substrates.

When the indicator moiety comprises alkaline phosphatase, then the detector moiety may comprise BCIP/NBT or p-nitrophenylphosphate. When the indicator moiety comprises CotA, the detector moiety may comprise naphthol, which changes from colorless to dark blue in the presence of CotA. When the indicator moiety comprises luciferase, the detector moiety may comprise luciferin, which undergoes a chemiluminescent reaction with luciferase.

It will be appreciated that the use of the apparatus according to the present invention may involve additional reaction steps in order to detect the desired analytes. For example, the cap may be removed from the shaft and treated with further reagents, or the cap may be treated with one or more reagents *in situ* on the shaft by passing the reagents through the hollow shaft.

In all aspects of the invention, the shaft(s) with the swab or biopsy punch attached may be sterilized, and may be packaged in a microorganism-impermeable container. The diagnostic caps may also be sterilized, but preferably they are not, because the caps do not come into contact with the patient bering diagnosed. It is an important advantage of the invention that the diagnostic caps do not need to be sterilized in the same way as the swab/biopsy punch. This feature greatly expands the range of diagnostic chemistry available, since a number of assay chemistries, such as antibody-based assays, can be degraded by the conditions used to sterilize medical articles.

Specific embodiments of the present invention will now be described further, hy way of example, with reference to the accompanying drawings, in which:

### BRIEF DESCRIPTION OF THE DRAWINGS

Figure 1 shows an apparatus according to the present invention comprising a swab, two diagnostic caps, and a syringe for introducing fluid into the swab shaft;
Figure 2 shows the embodiment of Figure 1 in longitudinal cross-section, with a diagnostic cap and the syringe secured on the swab shaft;
Figure 3 shows a longitudinal cross-section through an alternative embodiment comprising a biopsy punch;
Figure 4 shows a longitudinal cross-section through an embodiment of the diagnostic cap for use in the apparatus of the invention;
Figure 5 is a perspective view of a second diagnostic cap and swab system according to the present invention;
Figure 6 is a longitudinal cross-section through a cap and biopsy punch system according to the present invention before insertion of a biopsy punch into the sample receiving port of the cap;
Figure 7 is a longitudinal cross-section through the system of Fig.6 after insertion of the biopsy punch into the sample receiving port of the cap, and during the analysis using the device;
Figure 8 is a more detailed longitudinal cross-sectional view through the diagnostic cap of Fig.5;
Figure 9 is a bottom plan view of the diagnostic cap of Fig. 5;
Figure 10 is a bottom plan view of the diagnostic cap of Fig. 5 with the bottom housing part removed; and
Figure 11 is a schematic partial cross-section through one of the fluid flow paths in a second lateral flow diagnostic cap according to the present invention, similar to that of Fig.5, showing the different zones of the fluid flow path.

### DESCRIPTION OF THE PREFERRED EMBODIMENTS

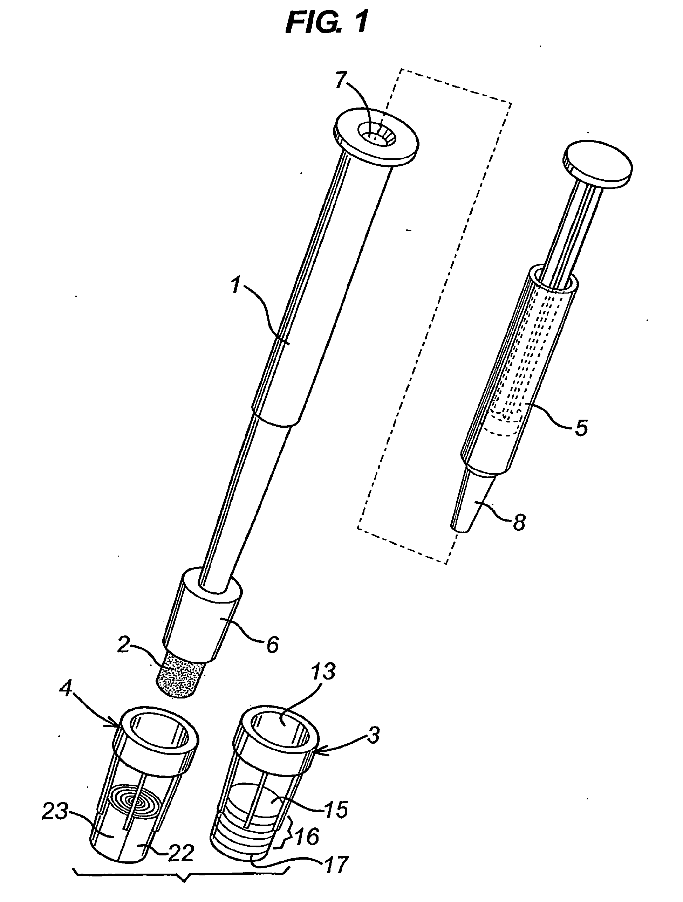

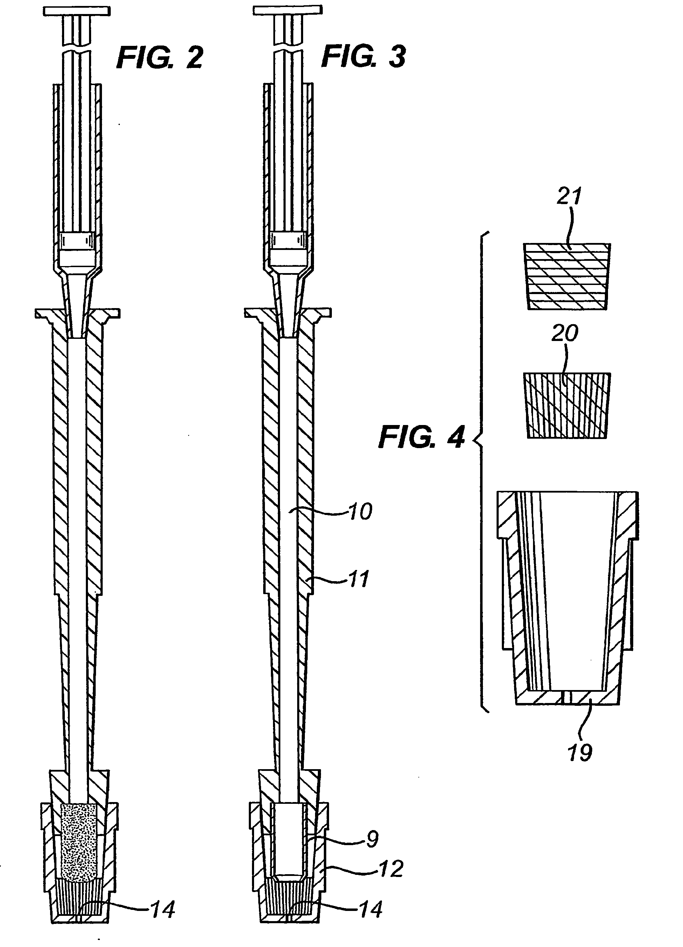

Referring to Figures 1 and 2, the apparatus comprises a hollow shaft 1, which is generally molded in one piece from thermoplastic polymer. A conventional swab 2 of medically acceptable sponge or nonwoven fibers is secured to a first end of the shaft 1, in fluid communication with the hollow interior of the shaft 1.

The apparatus further comprises two alternative diagnostic caps 3, 4. The caps 3, 4 each can form a snug interference fit with an expanded region 6 of the swab shaft. The expanded region 6 has a frusto-conical cross-section that is complementary to the internal cross-section of the openings of the caps 3, 4. In particular, it can be seen that the projection region 6 extends radially outwardly of the swab 2. As a result, quite small caps 3, 4 can be used in the apparatus according to the present invention.

At the top of the hollow shaft 1 is there is an opening 7 that can form a liquid-tight interference fit with the nozzle 8 of syringe 5. The syringe 5 may be filled with gas, and used simply to blow a liquid sample out of the swab 2 into the diagnostic cap 3, 4. Alternatively, the syringe 5 may contain water, saline or buffered saline to wash the sample out of the swab 2 into the cap 3, 4. In yet another embodiment, the syringe 5 may contain a diagnostic reagent in aqueous solution. In yet other embodiments, the apparatus may comprise a plurality of syringes 5 containing different aqueous solutions, for example different aqueous reagents for carrying out the desired analysis in the cap. It will be appreciated that the syringes 5 do not need to be sterile, since they are not applied to the patient.

Referring to Figure 3, the structure of the alternative embodiment is substantially identical to that of the embodiment shown in Figures 1 and 2, except that swab 2 has been replaced by a stainless-steel biopsy punch 9. In use, a homogeniser (not shown) may be passed down the bore 10 of shaft 11 to homogenise the biopsy punch sample before or after attachment of the diagnostic cap 12.

The structure of diagnostic cap 3 will now be described in more detail. The diagnostic cap 3 is formed by injection molding from a transparent polymer, such as polyethylene terephthalate (PET), polymethylmethacrylate (PMMA), or a transparent polyvinyl chloride (PVC). In certain preferred embodiments the cap is transparent both to UV radiation and 300-350 nanometers, and to visible radiation. This can be achieved, for example, with some transparent PVC plastics. The cap 3 has an opening 13 at the top that is shaped to form a liquid-tight interference fit on projection 6 of the swab shaft 1. A small gas-venting aperture 14 is provided in the bottom of cap 3 to assist the flow of the sample from the swab 2 into the cap 3. The lower part of the cap 3 is occupied by a stack of liquid-permeable disks 15, 16, 17. These disks include a filtration layer 15 for removing solid and cellular debris from the sample, diagnostic layers 16 each of which undergoes a color change in the presence of a different predetermined analyte, and a fill indicator layer 17, which undergoes a color change when wetted. The total amount of liquid required to wet all of the diagnostic layers 16 and the indicator layer 17 is only about 100 microliters, whereby little or no dilution of the sample collected on the swab is needed in order to carry out the diagnostic analysis for multiple analytes. Furthermore, it can be seen from Figures 2 and 3 that, in use, the diagnostic swab contacts the absorbent diagnostic layers in the cap 3, whereby a liquid sample in the swab can wick directly into those layers.

Diagnostic cap 4 comprises a transparent cap body similar to that of cap 3. However, instead of a stack of sensor disks 16 as shown in cap 3, cap 4 has a segmented annular diagnostic strip 20 inserted into the lower part thereof the annular strip 20 comprises a plurality of radially spaced stripes 22, 23 having sensitivity to different analytes in the sample. Color changes in these stripes 22, 23 are readable through the transparent sides of the cap. A plug 21 is inserted inside the annular diagnostic strip 20 to wick the fluid sample from the swab to the diagnostic strip 20. The plug 21 is made up of a bundle of hydrophilic polyester filaments aligned substantially coaxially with the cap. This bundle also serves to Filter solids and cellular debris front the sample before it reaches the diagnostic strip 20. The advantages of this arrangement are similar to those of cap 3, but even less of the relatively expensive diagnostic reagents are needed to form the strip 20. It is also possible that an even more compact cap suitable for even smaller fluid samples can be made by providing the plug 21 with a cylindrical cavity in the center thereof into which the swab 2 can be inserted.

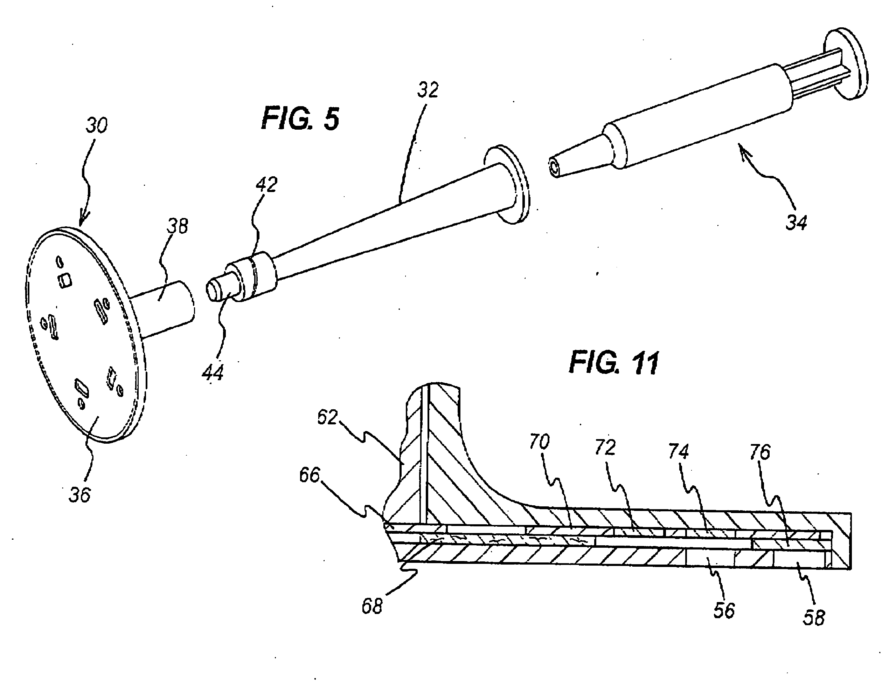

Referring to Figs. 5 to 8, the assay system comprises a lateral flow detection cap 30, a shaft 32 for biopsy punch 44, and a syringe 34. The detection cap 30 is generally disc-shaped, with a tubular sample receiving port 38 projecting upwardly from the center of the disc in a generally cylindrical formation. The shaft 32, biopsy punch 44, and syringe 34 are generally similar in construction to those in the embodiment of Fig. 1, except that the embodiments of Figs. 5 to 8 comprise complementary luer lock threads 42,46 on the enlarged region of the shaft and on the inside surface of the sample receiving port.

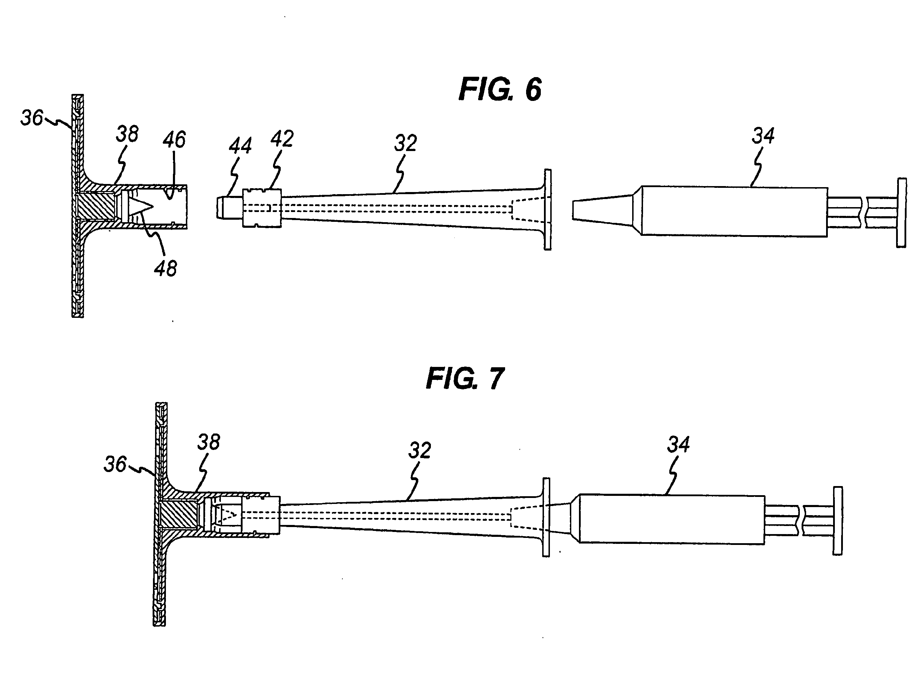

The sample receiving port in the embodiments shown in Figs. 6 and 7 is provided with a conical boss 18 projecting upwardly from the base of the port. In use, the conical boss 48 crushes the biopsy sample in the punch 44 to release liquid from the sample into the cap when the punch is secured onto the cap, as shown in Fig. 7.

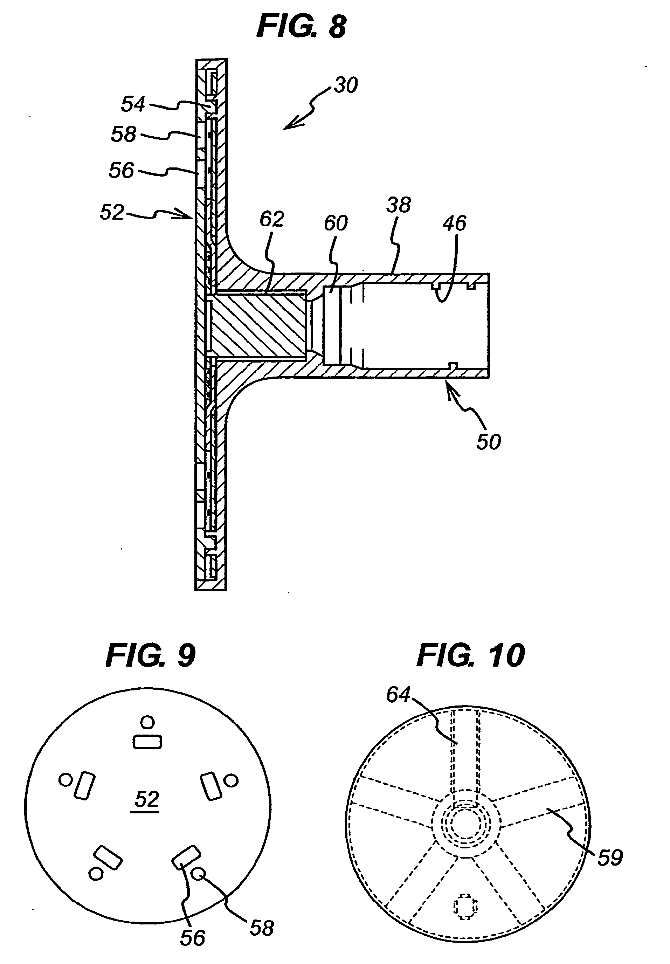

Referring to Figs. 8 to 10, the diagnostic cap 30 comprises a housing formed from an upper part 50 and a substantially disc-shaped lower part 52. Each part may be made by injection molding of thermoplastics. The upper housing part is itself made from two separate pieces. The upper and lower housing parts are fitted together by means of snap fittings 54. The lower surface of the upper housing part 50 comprises five radially spaced shallow recesses 59 for receiving respective test strips 64 of porous carrier materials, as shown in Fig. 10. Each test strip is adapted to test for a different analyte, for example analytes characteristic of a different wound pathology. The test strips 64 are retained in the recesses by the lower housing part 52. The fluid flow paths for the samples extend between the upper and lower housing parts along the recesses 59 when the lower housing part 52 and the upper housing part 50 are snap-fitted together.

As already noted, the upper housing part 50 comprises an upwardly projecting tubular sample receiving port 38 for receiving the swab. A filter membrane can be located in the bottom of the inlet port in region 60 for removing particles and cells from the sample, but in this embodiment the membrane is omitted in order to allow free passage to cell surface enzyme analytes. The sample then flows through a cylindrical (annular) inlet channel 62 to the upstream ends of the five test strips 64.

The lower housing part 52 comprises apertures for viewing the test strips. For each of the radially disposed test strips there is a detection window 56 above the detection zone of the strip, and a control window 58 above the end-of-assay indicator region of the test strip. The lower surface of the second housing part may further be provided with embossing or other indicia identifying the specific analyte detected in that strip. It will be appreciated that the windows 56,58 and optional indicia could alternatively be provided in the upper housing part 50 if viewing from the top of the device is preferred.

Referring to Figure 11, a schematic, enlarged cross-section perpendicular to one of the lateral flow paths of a device similar to that of Fig. 5 is shown. The flow path in this device contains a number of elements retained by the plastic housing. The first element in the flow path is a wicking pad 66 of non-woven textile material. This is located at the radially innermost end of the flow path, and positioned directly under the inlet channel 62 of the device. The wicking pad 66 is in capillary contact with reaction zone 68 of the flow path.

Reaction zone 68 is a porous glass fiber pad approximately 1 mm thick, 1cm wide, and 3cm long (i.e. extending about 3cm radially outward from the inlet 62). The glass fiber pad incorporates support beads having horseradish peroxidase (HRP) conjugated thereto by a peptide-containing linker as hereinbefore described. The peptide has 10-15 residues and contains the sequence ala-ala-pro-val that is specific to elastase.

Conjugation of HRP to the substrate-bound peptides can for example be achieved through the bi-functional crosslinking reagent sulfo-SMCC. The reaction is a two step process, including 1) formation of HRP-maleimide, followed by 2) reaction with a peptide that has been conjugated to the substrate surface using conventional methods.

Typical conditions for the HRP Conjugation as described by Mitchell C. Sanders *et al*. (op.cit.) are: (1) HRP - Maleimide conjugation: 2.5 mg of Roche HRP is dissolved in 500µl of 1M Na phosphate (pH of 7.4). Sulfo-SMCC is dissolved in 50µl DMSO, and combined with the HRP for 20 minutes at room temperature. Separation is accomplished with Gel filtration in maleimide conjugation buffer; (2) HRP-Peptide-Membrane Conjugation: The fractions that are free of sulfo-SMCC (∼1 ml) are combined with the substrate-bound peptide and reacted at ∼4°C overnight with rotation. Several washes are conducted, including two 20-minute washes with 100 mL of 0.1% triton in PBS, followed by two 20-minute washes with 0.1% PEG 5000 solution, and a 1 hr wash in 250 mL of a 10% sucrose solution, followed by speed vacuuming overnight.

An aliquot containing 10µl of a 10% solution of the conjugated beads is then pipetted onto the glass fiber strip to form the reaction zone.

The radially outermost end of the reaction zone pad 68 is in capillary contact with detection strip 70. Detection strip 70 is formed of a POREX membrane of total length about 4cm having deposited thereon a detection region 74 of 4-chloro-1-naphthol that undergoes a color change in the presence of HRP. The detection zone 74 is made by depositing 0.5µl of a 40mg/ml solution of 4-chloro-1-naphthol in ethanol onto a 1cm region of the detection strip.

The detection strip 70 is further provided with a control line 72 of nitrocellulose that reacts with a predetermined threshold amount of free HRP to reduce the incidence of false positive results. To form the control line 72, a solution of 2% nitrocellulose in methanol is made up and 0.5ul is deposited on a 1cm line, 1cm away from the test line.

Finally, the radially outward end of the detection strip 70 is in capillary contact with absorbent reservoir 76, which acts to draw the liquid sample through the test strip and capture the liquid at the end of the strip. The absorbent reservoir 76 is a nonwoven rayon/polyacrylate strip of length about 3cm that has been impregnated with a solution of 1% tetrabromophenol blue in distilled water and then dried. The indicator in the absorbent reservoir 76 changes color when it is soaked in the test solution, and this color change can be seen through window 58 of the housing. The color change can be used to confirm that a predetermined minimum volume of liquid sample has passed through the detection zone, thereby reducing the likelihood of false negative results.

In alternative embodiments, the swab may be replaced by a biopsy punch, optionally with means to macerate the sample.

In use, the swab is used to obtain a sample of wound fluid. Typically the sample will have volume about100µl of wound fluid. The swab is inserted into the sample receiving port of the device by means of the Luer lock. The sample is then expelled from the swab by injecting saline (sterile PBS) through the central channel of the swab from the syringe. The sample passes through the inlet channels and along the test strips. After a predetermined time, the underside of the device is viewed to assess the results of the analysis.

The above embodiments have been described by way of example only. Many other embodiments falling within the scope of the accompanying claims will be apparent to the skilled reader.

## Claims

1. A diagnostic test apparatus comprising:
a shaft (1,11,32) having a first end and a second end;
a swab (2) or a biopsy punch (9,44) mounted on the first end of the shaft; and
a cap (3,4,30) for fitting over the first end of the shaft, said cap containing at least
one diagnostic test reagent;
wherein the shaft (1,11,32) comprises at least one cap engagement element (6,42) extending radially outwardly of the swab (2) or the biopsy punch (9,44) for engagement with the cap (3,4,30) to retain the cap on the shaft (1,11,32), **characterized in that** said cap engagement element is located proximate to the first end from 1mm to 30 mm from the tip of the swab or biopsy punch in the direction of the second end, and wherein the shaft with the swab or biopsy punch attached is sterilised and said cap is not sterilised.

2. A diagnostic test apparatus according to claim 1, wherein the cap engagement element (6,42) is selected from the group consisting of: a tapered region of the shaft for forming an interference fit with the cap, a snap-fitting projection for forming a snap-fit with one or more complementary projections on an inner surface of the cap, and a thread projection for forming a screw fit with one or more complementary threads (46) on an inner surface of the cap.

3. A diagnostic test apparatus according to any preceding claim, wherein the shaft (1,11,32) is hollow, whereby a fluid can be passed down the shaft to expel a biological sample from the swab (2) or the biopsy punch (9,44).

4. A diagnostic test apparatus according to any preceding claim, wherein the complementary fitting elements (6,42) on the cap (3,4,30) and the shaft (1,11,32) are adapted to provide a substantially liquid-tight seal between the cap and the shaft.

5. A diagnostic test apparatus according to any preceding claim, wherein, when the cap (3,4,30) is secured on the shaft (1,11,32), the swab or the biopsy is in contact with an absorbent material (21) that can wick fluid from the swab or the biopsy to the one or more diagnostic reagents.

6. A diagnostic test apparatus according to any preceding claim, wherein a swab (2) is attached to the first end of the shaft (1) and the sample receiving port of the cap (3) is dimensioned such that, when the cap (3) is secured on the shaft (1), the swab is compressed by the diagnostic cap to squeeze fluid out of the swab.

7. A diagnostic test apparatus according to any preceding claim, wherein a biopsy punch (9,44) is attached to the first end of the shaft (11,32) and the cap further comprises an internal projection (48) adapted to crush or macerate a tissue sample in the biopsy punch when the punch (9,44) is secured on the cap.

8. A diagnostic test apparatus according to any preceding claim, wherein the cap (3,4,30) is at least partially transparent, or the cap (30) comprises one or more window openings (56,58) therein, to permit visual observation of a diagnostic indicator inside the cap.

9. A diagnostic test apparatus according to any of claims 1 to 8, wherein the cap is a diagnostic cap (3,4) comprising a substantially cup-shaped body, an absorbent plug (21) located within the body, and at least one diagnostic test reagent located in or around the absorbent plug.

10. A diagnostic test apparatus according to claim 9, wherein the at least one diagnostic test reagent is provided in or on an annular diagnostic strip (20) extending radially around the inside of the cap.

11. A diagnostic test apparatus according to claim 9, wherein the at least one diagnostic test reagent is provided in or on a diagnostic sheet (15,16,17) extending transversely across the inside of the cap (3).

12. A diagnostic test apparatus according to claim 9, wherein the absorbent plug (21) has an uncompressed volume of from about 10 to about 1000mm³, preferably from about 50 to about 300 mm³.

13. A diagnostic test apparatus according to any of claims 1 to 8, wherein the cap (30) comprises a sample receiving port (38) having an interior surface configured for engagement with the cap engagement element (42) on the shaft, and a housing defining a lateral flow path for a liquid sample, the lateral flow path having an inlet end in fluid communication with the sample receiving port (38).

14. A diagnostic test apparatus according to claim 13, wherein the housing further comprises a base for the cap (30), whereby the cap can rest on said base on a horizontal surface with the sample receiving port (38) opening in an upward direction to receive the shaft.

15. A diagnostic test apparatus according to claim 14, wherein the housing has a substantially flat lower surface forming said base for the housing, and the sample receiving port (38) opens upwardly from an upper surface of the housing opposite said flat lower surface.

16. A diagnostic test apparatus according to claim 14 or 15, wherein the lateral flow path extends substantially in a plane that is substantially perpendicular to the axis of the sample receiving port (38).

17. A diagnostic test apparatus according to any of claims 14 to 16, wherein the housing contains a plurality of lateral flow paths for detecting a plurality of different analytes.

18. A diagnostic test apparatus according to claim 17, wherein the plurality of lateral flow paths are spaced radially around the sample receiving port (38), preferably in a plane substantially perpendicular to the axis of the sample receiving port (38).

19. A diagnostic test apparatus according to any preceding claim, comprising both a first said shaft (1) having a swab attached thereto, and a second said shaft (11,32) having a biopsy punch attached thereto, and wherein the diagnostic cap (3,4,30) can be secured interchangeably on the said first and second shafts.

## Patentansprüche

1. Diagnostisches Testgerät, das Folgendes umfasst:
einen Schaft (1, 11, 32) mit einem ersten Ende und einem zweiten Ende;
ein Tupfer (2) oder eine Biopsiestanze (9, 44), die auf dem ersten Ende des Schafts befestigt ist; und
eine Kappe (3, 4, 30) zum Aufsetzen auf das erste Ende des Schafts, wobei die Kappe zumindest ein diagnostisches Testreagenz enthält;
worin der Schaft (1, 11, 32) zumindest ein Kappeneingriffselement (6, 42) umfasst, das sich vom Tupfer (2) oder der Biopsiestanze (9, 44) zum Eingriff mit der Kappe (3, 4, 30) radial nach außen erstreckt, um die Kappe auf dem Schaft (1, 11, 32) zu halten,
**dadurch gekennzeichnet, dass** das Kappeneingriffselement in der Nähe des ersten Endes 1 mm bis 30 mm von der Spitze des Tupfers oder der Biopsiestanze entfernt zum zweiten Ende hin angeordnet ist, und worin der Tupfer Schaft mit dem oder der Biopsiestanze sterilisiert ist und die Kappe nicht sterilisiert ist.

2. Diagnostisches Testgerät nach Anspruch 1, worin das Kappeneingriffselement (6, 42) aus der folgenden Gruppe ausgewählt ist: eine verjüngte Region des Schafts zur Bildung einer Interferenzpassung mit der Kappe, ein Rastvorsprung zur Bildung einer Rastverbindung mit einem oder mehr komplementären Vorsprüngen auf einer Innenseite der Kappe, und ein Gewindevorsprung zur Bildung einer Schraubenverbindung mit einem oder mehr komplementären Gewinden (46) auf einer Innenseite der Kappe.

3. Diagnostisches Testgerät nach einem der vorhergehenden Ansprüche, worin der Schaft (1, 11, 32) hohl ist, wodurch eine Flüssigkeit durch den Schaft gespült werden kann, um eine biologische Probe vom Tupfer (2) oder der Biopsiestanze (9, 44) zu drücken.

4. Diagnostisches Testgerät nach einem der vorhergehenden Ansprüche, worin die komplementären Passelemente (6, 42) auf der Kappe (3, 4, 30) und dem Schaft (1, 11, 32) eine im Wesentlichen flüssigkeitsdichte Abdichtung zwischen der Kappe und dem Schaft bereitstellen können.

5. Diagnostisches Testgerät nach einem der vorhergehenden Ansprüche, worin der Tupfer oder die Biopsie mit einem saugfähigen Material (21), das Flüssigkeit aus dem Tupfer oder der Biopsie zu den ein oder mehr diagnostischen Reagenzien leiten kann, in Berührung ist, wenn die Kappe (3, 4, 30) auf dem Schaft (1, 11, 32) befestigt ist.

6. Diagnostisches Testgerät nach einem der vorhergehenden Ansprüche, worin ein Tupfer (2) am ersten Ende des Schafts (1) befestigt ist und die Probenaufnahmeöffnung der Kappe (3) solche Abmessungen aufweist, dass der Tupfer zum Herausdrücken von Flüssigkeit aus dem Tupfer von der diagnostischen Kappe zusammengedrückt wird, wenn die Kappe (3) auf dem Schaft (1) befestigt ist.

7. Diagnostisches Testgerät nach einem der vorhergehenden Ansprüche, worin eine Biopsiestanze (9, 44) am ersten Ende des Schafts (11, 32) befestigt ist und die Kappe ferner einen inneren Vorsprung (48) umfasst, der eine Gewebeprobe in der Biopsiestanze zerdrücken oder mazerieren kann, wenn die Stanze (9, 44) auf der Kappe befestigt ist.

8. Diagnostisches Testgerät nach einem der vorhergehenden Ansprüche, worin die Kappe (3, 4, 30) zumindest teilweise transparent ist oder worin die Kappe (30) eine oder mehr Fensteröffnungen (56, 58) darin umfasst, um die visuelle Beobachtung eines diagnostischen Indikators in der Kappe zu ermöglichen.

9. Diagnostisches Testgerät nach einem der Ansprüche 1 bis 8, worin die Kappe eine diagnostische Kappe (3, 4) ist, die einen im Wesentlichen tassenförmigen Körper, einen im Körper angeordneten saugfähigen Pfropf (21) und zumindest ein in oder um den saugfähigen Pfropf angeordnetes diagnostisches Testreagenz umfasst.

10. Diagnostisches Testgerät nach Anspruch 9, worin das zumindest eine diagnostische Testreagenz in oder auf einem ringförmigen Diagnosestreifen (20) vorgesehen ist, der sich radial um die Innenseite der Kappe erstreckt.

11. Diagnostisches Testgerät nach Anspruch 9, worin das zumindest eine diagnostische Testreagenz in oder auf einer Diagnoseplatte (15, 16, 17) vorgesehen ist, die sich quer über die Innenseite der Kappe (3) erstreckt.

12. Diagnostisches Testgerät nach Anspruch 9, worin der saugfähige Pfropf (21) ein unkomprimiertes Volumen von ca. 10 bis ca. 1000 mm³, vorzugsweise von ca. 50 bis ca. 300 mm³ aufweist.

13. Diagnostisches Testgerät nach einem der Ansprüche 1 bis 8, worin die Kappe (30) eine Probenaufnahmeöffnung (38) mit einer zum Eingriff mit dem Kappeneingriffselement (42) auf dem Schaft ausgelegten Innenseite und ein einen seitlichen Strömungspfad für eine Flüssigkeitsprobe definierendes Gehäuse umfasst, wobei der seitliche Strömungspfad ein Einlassende aufweist, das mit der Probenaufnahmeöffnung (38) in Flüssigkeitsverbindung steht.

14. Diagnostisches Testgerät nach Anspruch 13, worin das Gehäuse ferner einen Boden für die Kappe (30) umfasst, wodurch die Kappe auf dem Boden auf einer horizontalen Fläche ruhen kann, wobei sich die Probenaufnahmeöffnung (38) nach oben öffnet, um den Schaft aufzunehmen.

15. Diagnostisches Testgerät nach Anspruch 14, worin das Gehäuse eine im Wesentlichen flache Unterseite aufweist, welche den Boden für das Gehäuse bildet, und wobei sich die Probenaufnahmeöffnung (38) von einer Oberseite des Gehäuses gegenüber der flachen Unterseite nach oben öffnet.

16. Diagnostisches Testgerät nach Anspruch 14 oder 15, worin sich der seitliche Strömungspfad im Wesentlichen in einer Ebene erstreckt, die im Wesentlichen senkrecht zur Achse der Probenaufnahmeöffnung (38) ist.

17. Diagnostisches Testgerät nach einem der Ansprüche 14 bis 16, worin das Gehäuse zum Nachweis einer Vielzahl verschiedener Analyten eine Vielzahl von seitlichen Strömungspfaden enthält.

18. Diagnostisches Testgerät nach Anspruch 17, worin die Vielzahl von seitlichen Strömungspfaden radial im Abstand um die Probenaufnahmeöffnung (38) angeordnet sind, vorzugsweise in einer Ebene, die im Wesentlichen senkrecht zur Achse der Probenaufnahmeöffnung (38) ist.

19. Diagnostisches Testgerät nach einem der vorhergehenden Ansprüche, das einen ersten Schaft (1) mit einem daran befestigten Tupfer und einen zweiten Schaft (11, 32) mit einer daran befestigten Biopsiestanze umfasst, und worin die Diagnosekappe (3, 4, 30) auswechselbar an den ersten und zweiten Schäften befestigt werden kann.

## Revendications

1. Appareil de tests de diagnostic comportant :
une tige (1, 11, 32) présentant une première extrémité et une seconde extrémité ;
un badigeon (2) ou un emporte-pièce (9, 44) de biopsie monté sur la première extrémité de la tige ; et
un capuchon (3, 4, 30) destiné à être monté sur la première extrémité de la tige, ledit capuchon contenant au moins un réactif de test de diagnostic ;
dans lequel la tige (1, 11, 32) comporte au moins un élément (6, 42) d'accouplement du capuchon s'étendant radialement vers l'extérieur du badigeon (2) ou de l'emporte-pièce (9, 44) de biopsie pour s'accoupler avec le capuchon (3, 4, 30) afin de retenir le capuchon sur la tige (1, 11, 32), **caractérisé en ce que** ledit élément d'accouplement du capuchon est situé à proximité de la première extrémité à une distance de 1 à 30 mm depuis le bout du badigeon ou de l'emporte-pièce de biopsie dans la direction de la seconde extrémité, et dans lequel la tige avec le badigeon ou l'emporte-pièce de biopsie attaché est stérilisée et ledit capuchon n'est pas stérilisé.

2. Appareil de tests de diagnostic selon la revendication 1, dans lequel l'élément (6, 42) d'accouplement du capuchon est choisi dans le groupe constitué d'une région évasée de la tige pour former un ajustement serré avec le capuchon, une saillie de raccord à encliquetage pour former un raccord encliquetable avec une ou plusieurs saillies complémentaires sur une surface interne du capuchon, et une saillie filetée pour former un ajustement à vis avec un ou plusieurs filets (46) complémentaires sur une surface interne du capuchon.

3. Appareil de tests de diagnostic selon l'une quelconque des revendications précédentes, dans lequel la tige (1, 11, 32) est creuse, selon lequel on peut faire descendre un fluide le long de la tige pour expulser un échantillon biologique depuis le badigeon (2) ou l'emporte-pièce (9, 44) de biopsie.

4. Appareil de tests de diagnostic selon l'une quelconque des revendications précédentes, dans lequel les éléments (6, 42) de raccord à encliquetage complémentaires sur le capuchon (3, 4, 30) et la tige (1, 11, 32) sont conçus pour fournir une étanchéité sensiblement totale aux liquides entre le capuchon et la tige.

5. Appareil de tests de diagnostic selon l'une quelconque des revendications précédentes, dans lequel, lorsque le capuchon (3, 4, 30) est fixé sur la tige (1, 11, 32), le badigeon ou la biopsie touche un matériau (21) absorbant qui peut drainer le fluide depuis le badigeon ou la biopsie jusqu'à un ou plusieurs réactifs de diagnostic.

6. Appareil de tests de diagnostic selon l'une quelconque des revendications précédentes, dans lequel un badigeon (2) est attaché à la première extrémité de la tige (1) et l'orifice de réception d'échantillon du capuchon (3) est dimensionné de telle sorte que, lorsque le capuchon (3) est fixé à la tige (1), le badigeon est comprimé par le capuchon de diagnostic pour forcer le fluide hors du badigeon.

7. Appareil de tests de diagnostic selon l'une quelconque des revendications précédentes, dans lequel un emporte-pièce pour biopsie (9, 44) est attaché à la première extrémité de la tige (11, 32) et le capuchon comporte en outre une saillie (48) interne conçue pour écraser ou faire macérer un échantillon de tissu cellulaire dans l'emporte-pièce de biopsie lorsque l'emporte-pièce (9, 44) est fixé sur le capuchon.

8. Appareil de tests de diagnostic selon l'une quelconque des revendications précédentes, dans lequel le capuchon (3, 4, 30) est au moins partiellement transparent, ou le capuchon (30) comporte une ou plusieurs ouvertures (56, 58) de fenêtre aménagées dans ce dernier, pour permettre une observation visuelle d'un indicateur diagnostique à l'intérieur du capuchon.

9. Appareil de tests de diagnostic selon l'une quelconque des revendications 1 à 8, dans lequel le capuchon est un capuchon (3, 4) de diagnostic comportant un corps sensiblement en forme de godet, un bouchon (21) absorbant situé à l'intérieur du corps, et au moins un réactif de test de diagnostic situé dans le bouchon absorbant ou autour de ce dernier.

10. Appareil de tests de diagnostic selon la revendication 9, dans lequel ledit au moins un réactif de test de diagnostic est fourni dans ou sur une bandelette (20) de diagnostic annulaire s'étendant radialement autour de l'intérieur du capuchon.

11. Appareil de tests de diagnostic selon la revendication 9, dans lequel ledit au moins un réactif de test de diagnostic est fourni dans ou sur une feuille (15, 16, 17) de diagnostic s'étendant transversalement dans l'intérieur du capuchon (3).

12. Appareil de tests de diagnostic selon la revendication 9, dans lequel le bouchon (21) absorbant a un volume non comprimé allant d'environ 10 à environ 1000 mm³, de préférence d'environ 50 à environ 300 mm³,

13. Appareil de tests de diagnostic selon l'une quelconque des revendications 1 à 8, dans lequel le capuchon (30) comporte un orifice (38) de réception d'échantillon présentant une surface intérieure configurée pour s'accoupler avec l'élément (42) d'accouplement du capuchon sur la tige, et un logement définissant une voie d'écoulement latérale pour un échantillon liquide, la voie d'écoulement latérale présentant une extrémité d'orifice d'entrée en communication fluidique avec l'orifice (38) de réception d'échantillon.

14. Appareil de tests de diagnostic selon la revendication 13, dans lequel le logement comporte en outre une base pour le capuchon (30), selon lequel le capuchon peut reposer sur ladite base sur une surface horizontale avec l'orifice (38) de réception d'échantillon s'ouvrant dans une direction vers le haut pour recevoir la tige.

15. Appareil de tests de diagnostic selon la revendication 14, dans lequel le logement présente une surface inférieure sensiblement plane formant ladite base pour le logement, et l'orifice (38) de réception d'échantillon s'ouvre vers le haut depuis une surface supérieure du logement opposée à ladite surface inférieure plane.

16. Appareil de tests de diagnostic selon la revendication 14 ou 15, dans lequel la voie d'écoulement latérale s'étend sensiblement dans un plan qui est sensiblement perpendiculaire à l'axe de l'orifice (38) de réception d'échantillon.

17. Appareil de tests de diagnostic selon l'une quelconque des revendications 14 à 16, dans lequel le logement contient plusieurs voies d'écoulement latérales pour détecter plusieurs analytes différents.

18. Appareil de tests de diagnostic selon la revendication 17, dans lequel lesdites plusieurs voies d'écoulement latérales sont espacées radialement autour de l'orifice (38) de réception d'échantillon, de préférence dans un plan sensiblement perpendiculaire à l'axe de l'orifice (38) de réception d'échantillon.

19. Appareil de tests de diagnostic selon l'une quelconque des revendications précédentes, comportant à la fois une dite première tige (1) pourvue d'un badigeon qui y est attaché, et une dite seconde tige (11, 32) pourvue d'un emporte-pièce de biopsie qui y est attaché, et dans lequel le capuchon (3, 4, 30) de diagnostic peut être fixé de manière interchangeable sur lesdites première et seconde tiges.
